# EUROPEAN PATENT APPLICATION

(11) **EP 2 482 064 A1**
(43) Date of publication of application: **01.08.2012**
(21) Application number: 10818720.4
(22) Date of filing: 14.09.2010
(51) Int. Cl.: G01N 27/62, G01N 33/66

(54) **METHOD FOR DETERMINATION OF RENAL CELL CARCINOMA**

(30) Priority: 25.09.2009 JP 2009220893
(71) Applicant: National University Corporation Hokkaido University, Sapporo-shi, Hokkaido 060-0808 (JP); Hirosaki University, Hirosaki-shi, Aomori 036-8560 (JP); Shionogi & Co., Ltd., Osaka 541-0045 (JP)
(72) Inventor: NISHIMURA, Shin-Ichiro, Hokkaido 060-0808 (JP); MIURA, Yoshiaki, Hokkaido 060-0808 (JP); NAKAHARA, Taku, Hokkaido 060-0808 (JP); AMANO, Maho, Hokkaido 060-0808 (JP); OHYAMA, Chikara, Hirosaki-shi Aomori 036-8560 (JP); HATAKEYAMA, Shingo, Hirosaki-shi Aomori 036-8560 (JP)
(74) Representative: Vossius & Partner
(86) International application number: PCT/JP2010/065842
(87) International publication number: WO 2011/037045

(57) **Abstract**

The present invention provides a novel method for diagnosing renal cell carcinoma. The method of the invention allows evaluation of the presence of renal cell carcinoma or the degree of malignancy of renal cell carcinoma, by conducting comprehensive analysis of N-linked sugar chains in serum of a patient and utilizing an expression level of detected sugar chain as an index of the evaluation.

## Description

### TECHNICAL FIELD

The present invention relates to a method for determination of renal cell carcinoma. In particular, the present invention relates to a method for determination of the presence of renal cell carcinoma and a method for determination of the degree of malignancy of renal cell carcinoma.

### BACKGROUND ART

Renal carcinoma accounts for 2-3% of all tumors in adults, and renal cell carcinoma represents 90-95% of renal carcinoma. With the developments in diagnostic imaging technology, there is an increasing number of cases where renal carcinoma is found in a relatively early stage. However, renal carcinoma often develops without symptoms until it reaches large enough size, and there is more than 30 percent of the cases where renal carcinoma is found as advanced carcinoma. In 2006 in the United States, 36,000 people were newly diagnosed with renal carcinoma, and 13,000 people died. It is difficult to find renal carcinoma at an early stage because effective organ-specific tumor marker has not been identified. Furthermore, effective predictive marker for prognosis of renal cell carcinoma has not been identified, and the difficulty in assessing need of postoperative supplemental therapy and indication of surgery contributes to high mortality.

Recently, methods for diagnosis of renal carcinoma have been reported. For example, JP-A-2008-209369 (Patent Document 1) discloses determination of protein expression in renal carcinoma tissue. JP-A-2007-267700 (Patent Document 2) discloses detection of methylation of anti-oncogene in renal cell carcinoma tissue. JP-A-2004-77268 (Patent Document 3) discloses detection and determination of the presence of a peptide that inhibits angiogenesis in cancer tissue. Tunuguntla, H.S.G.R. et al., The Journal of Urology 179: 2096-2102 (2008) (Non-Patent Document 1) discloses a method for determination of glycoproteins in serum. Baldewijins, M.M.L. et al., Biochimicaet Biophysica Acta 1785: 133-155 (2008) (Non-Patent Document 2) describes genetic diagnosis by detecting oncogene. JP-A-2009-506307 (Patent Document 4) describes diagnosis of renal cell carcinoma by determining the level of immunoreaction against matrix metalloproteinase (MMP)-7 or the level of anti MMP-7 antibody. Sandim V., et al., "Renal Cell Carcinoma and Proteomics", Urologia Internationalis 84:373-377 (2010) (Non-Patent Document 3) is a review of proteome analysis of renal cell carcinoma and describes that further developments in the proteome analysis technology of renal cell carcinoma are needed for search of a biomarker that enables early detection of renal cell carcinoma.

[Patent Document 1] JP-A-2008-209369
[Patent Document 2] JP-A-2007-267700
[Patent Document 3] JP-A-2004-77268
[Patent Document 4] JP-A-2009-506307

[Non-Patent Document 1] Tunuguntla, H.S.G.R. et al., The Journal of Urology 179: 2096-2102 (2008)
[Non-Patent Document 2] Baldewijins, M.M.L. et al., Biochimicaet Biophysica Acta 1785: 133-155 (2008)
[Non-Patent Document 3] Sandim V., et al., "Renal Cell Carcinoma and Proteomics", Urologia Internationalis 84:373-377 (2010)

### DISCLOSURE OF INVENTION

### PROBLEMS TO BE SOLVED BY THE INVENTION

Although there are reports regarding diagnostic imaging technology of renal carcinoma as mentioned above, effective marker enabling early detection and diagnosis of renal cell carcinoma has not been identified. Renal cell carcinoma in an early stage has often no symptoms, and therefore, it is a form of cancer that is hard to detect in an early stage. There is a report that about 40 percent of patients had advanced or metastatic carcinoma at the time of definitive diagnosis. Such patients have a less favorable prognosis as low as less than 10 percent of five year survival rate, and thus, there is need for effective marker enabling specific detection of renal carcinoma in an early stage.
Furthermore, it is important to predict how long a patient will keep well after diagnosed as being carcinoma, in view of quality-of-life (QOL) of and effective treatment for the patient. Accordingly, there is also need for a marker enabling prediction of the degree of malignancy of renal carcinoma, in particular, enabling diagnosis and prognostic prediction of advanced renal cell carcinoma.

Although there has been an increase in a research for diagnostic method for renal cell carcinoma by targeting a protein expression in a carcinoma-specific manner, most of them remain in an analysis at a genetic level and make no further progress. For applying such analysis at a genetic level to clinical practice, there are many problems unsolved, such as cost and time issues. Furthermore, in direct detection of a protein expression in a carcinoma-specific manner, there are many issues that need to be improved, such as occurrence of a false-positive and a false-negative, insufficient detection sensitivity. Additionally, test methods as conventionally used, such as MRI, CT, angiography, are not practical for use in primary screening because they require expensive apparatus and cannot test many people at once.

### MEANS FOR SOLVING THE PROBLEM

The inventors have conducted comprehensive analysis of N-linked sugar chains in serum and discovered a sugar chain structure useful as a biomarker that correlates with the presence or the degree of malignancy of renal cell carcinoma. That is, the inventors have discovered that the presence or the degree of malignancy of renal cell carcinoma can be determined with sufficient specificity and sensitivity by measuring the abundance of certain sugar chain or of a plurality of certain sugar chains in a subject's blood (serum), using a quantitative high-speed and comprehensive sugar chain enrichment technology (glycoblotting method), which was developed by the inventors [Nishimura et al. (Nishimura S.-I., Niikura K., Kurogochi M., Matsushita T., Fumoto M., Hinou H., Kamitani R., Nakagawa H., Deguchi K., Miura N., Monde K., Kondo H.), "High-Throughput Protein Glycomics: Combined Use of Chemoselective Glycoblotting and MALDI-TOF/TOF Mass Spectrometry", Angew. Chem. Int. Ed., 44, 91-96 (2005)].

The present invention provides the followings.
(1) A method for determining the presence of renal cell carcinoma in a patient comprising the following steps:
   a) collecting all N-linked sugar chains from serum of the patient;
   b) obtaining a quantitive profile of said N-linked sugar chains;
   c) calculating an expression level of one sugar chain as detected, a ratio of expression level of one sugar chain to another sugar chain as detected or a summation of expression levels of sugar chains as detected; and
   d) determining the presence of renal cell carcinoma utilizing said expression level, said ratio of expression level or said summation of expression levels as an index of the determination.
(2) The method according to (1) wherein the sugar chain(s) in step (c) is selected from the group consisting of RC1, RC5, RC15, RC19, RC22, RC23, RC25, RC32, RC33, RC47 and a sugar chain having a tetra-antennary structure or a tri-antennary and bisecting structure.
(3) The method according to (1) wherein an expression level of RC33 or RC19 is calculated in step (c).
(4) The method according to (1) wherein a ratio of expression level of RC33 to RC15 (RC33/RC15), RC33 to RC1 (RC33/RC1), RC47 to RC15 (RC47/RC15), RC33 to RC22 (RC33/RC22), RC19 to RC15 (RC19/RC15), RC19 to RC32 (RC19/RC32), RC1 to RC19 (RC1/RC19), RC33 to RC5 (RC33/RC5), RC23 to RC5 (RC23/RC5) or RC33 to RC25 (RC33/RC25) is calculated in step (c).
(5) The method according to (1) wherein a summation of expression levels of sugar chains having a tetra-antennary structure or a tri-antennary and bisecting structure is calculated in step (c).
(6) A method for determining the degree of malignancy of renal cell carcinoma in a patient comprising the following steps:
   a) collecting all N-linked sugar chains from serum of the patient;
   b) obtaining a quantitive profile of said N-linked sugar chains;
   c) calculating a ratio of expression level of one sugar chain to another sugar chain as detected; and
   d) determining the degree of malignancy of renal cell carcinoma utilizing said ratio of expression level as an index of the determination.
(7) The method according to (6) wherein the sugar chain(s) in step (c) is selected from the group consisting of RC2, RC3, RC4, RC5, RC6, RC7, RC8, RC9, RC12, RC13, RC17, RC18, RC19, RC29, RC31 and RC40.
(8) The method according to (6) or (7) wherein the degree of malignancy of renal cell carcinoma is determined as to whether it is advanced renal cell carcinoma or not.
(9) The method according to (8) wherein a ratio of expression level of RC31 to RC29 (RC31/RC29), RC9 to RC6 (RC9/RC6) or RC4 to RC6 (RC4/RC6) is calculated in step (c).
(10) The method according to (6) or (7) wherein the degree of malignancy of renal cell carcinoma is determined as to the prognosis of said renal cell carcinoma.
(11) The method according to (10) wherein a ratio of expression level of RC3 to RC6 (RC3/RC6), RC7 to RC8 (RC7/RC8), RC2 to RC8 (RC2/RC8), RC17 to RC18 (RC17/RC18), RC12 to RC13 (RC12/RC13), RC3 to RC8 (RC3/RC8), RC5 to RC29 (RC5/RC29), RC5 to RC40 (RC5/RC40) or RC19 to RC5 (RC19/RC5) is calculated in step (c).
(12) Use of a sugar chain selected from the group consisting of RC1, RC5, RC15, RC19, RC22, RC23, RC25, RC32, RC33 and RC47 or a sugar chain having a tetra-antennary structure or a tri-antennary and bisecting structure as a diagnostic marker of renal cell carcinoma.
(13) Use according to (12) wherein the selected sugar chain is RC33 or RC19.
(14) Use according to (12) wherein the sugar chains are selected as the combination of RC33 and RC15, RC33 and RC1, RC47 and RC15, RC33 and RC22, RC19 and RC15, RC19 and RC32, RC1 and RC19, RC33 and RC5, RC23 and RC5 or RC33 and RC25.
(15) Use according to (12) wherein the selected sugar chain is a sugar chain having a tetra-antennary structure or a tri-antennary and bisecting structure.
(16) Use of a sugar chain selected from the group consisting of RC2, RC3, RC4, RC6, RC7, RC8, RC9, RC12, RC13, RC17, RC18, RC29 and RC31 as a diagnostic marker of advanced renal cell carcinoma.
(17) Use according to (16) wherein the sugar chains are selected as a combination of RC31 and RC29, RC9 and RC6 or RC4 and RC6.
(18) Use of a sugar chain selected from the group consisting of RC2, RC3, RC4, RC5, RC6, RC7, RC8, RC9, RC12, RC13, RC17, RC18, RC19, RC29, RC31 and RC40 as a predictive marker for prognosis of renal cell carcinoma.
(19) Use according to (18) wherein the sugar chains are selected as the combination of RC3 and RC6, RC7 and RC8, RC2 and RC8, RC17 and RC18, RC12 and RC13, RC3 and RC8, RC5 and RC29, RC5 and RC40 or RC19 and RC5.

### EFFECT OF THE INVENTION

The present invention enables determination of the presence or the degree of malignancy of renal cell carcinoma with greater sensitivity and specificity than that of conventional methods. Furthermore, in the method of the invention, a sugar chain analysis is conducted using a blood sample from a patient, and therefore, the patient's burden is extremely low. Additionally, the method of the invention allows multiple determinations by selecting relevant sugar chain(s) for use in such determination from the results of sugar chain analysis obtained in a single measurement, and thus, frequent testing is not necessary. Additionally, the present invention allows testing of many people at once and at a low cost compared to other methods such as genetic diagnosis.

### BRIEF DESCRIPTION OF THE DRAWINGS

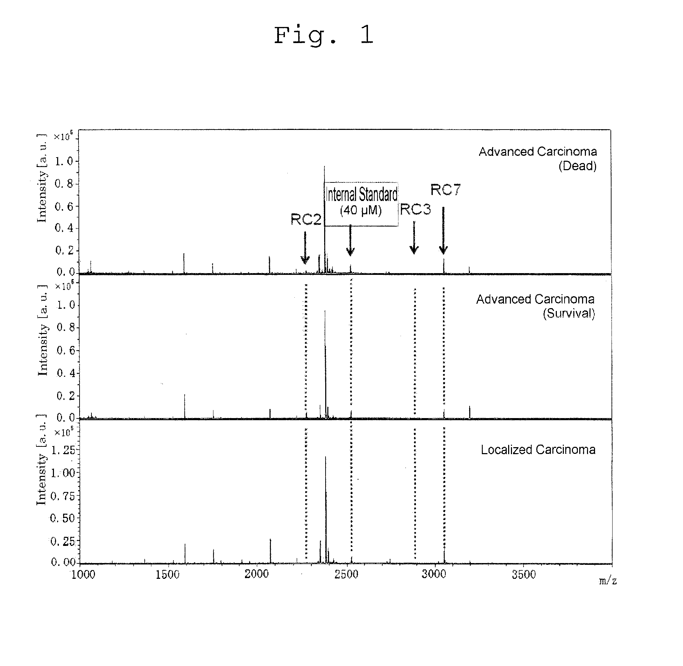

[Figure 1] A MALDI-TOF MS spectrum of N-linked sugar chains in serum from renal cell carcinoma patients. The upper panel: advanced carcinoma (dead case); the middle panel: advanced carcinoma (survival case); the lower panel: localized carcinoma.

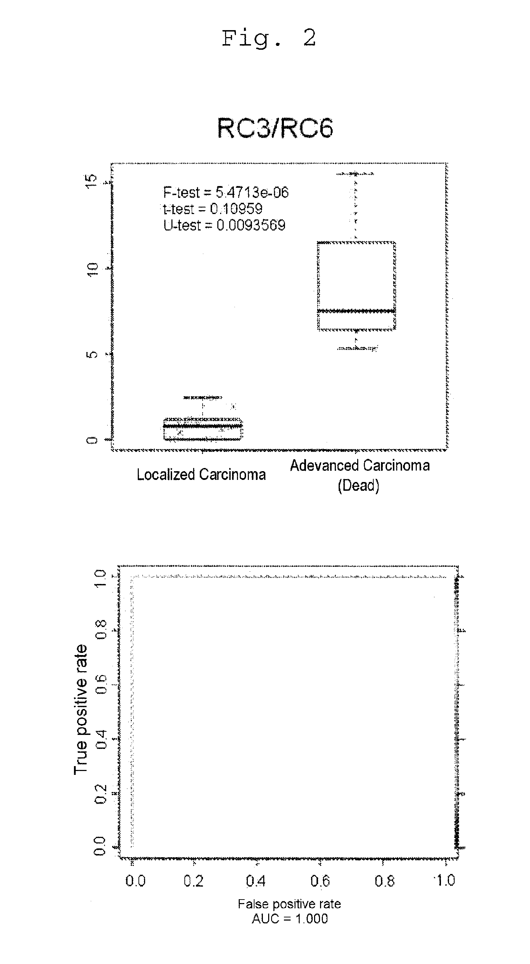

[Figure 2] The upper panel: a boxplot showing distribution of the ratio of expression level of RC3 to RC6 (RC3/RC6) in serum from renal cell carcinoma patients (localized carcinoma and advanced carcinoma (dead case)). The lower panel: a ROC curve of the ratio of expression level of RC3 to RC6 (RC3/RC6) (vertical line: true positive rate; horizontal line: false positive rate).

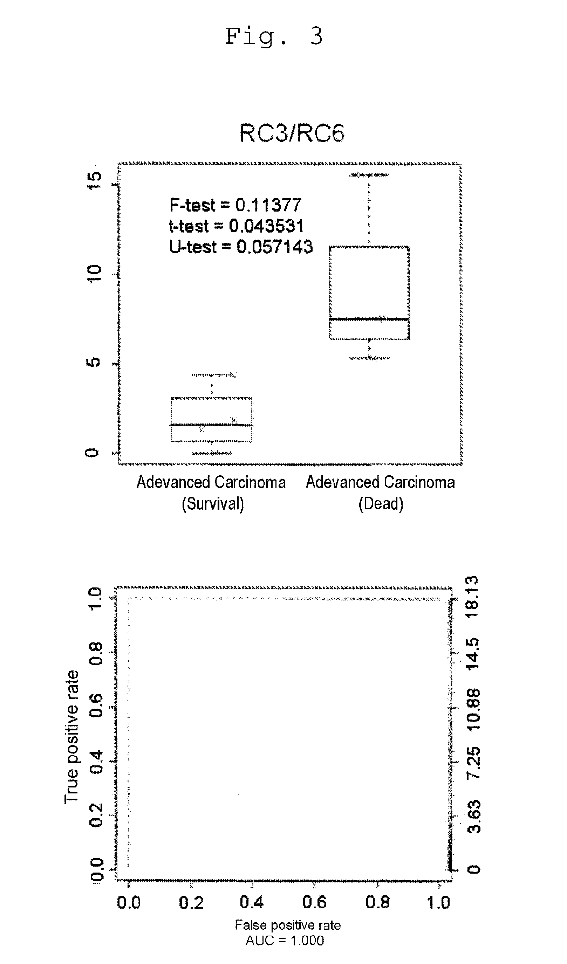

[Figure 3] The upper panel: a boxplot showing distribution of the ratio of expression level of RC3 to RC6 (RC3/RC6) in serum from renal cell carcinoma patients (advanced carcinoma (survival case) and advanced carcinoma (dead case)). The lower panel: a ROC curve of the ratio of expression level of RC3 to RC6 (RC3/RC6) (vertical line: true positive rate; horizontal line: false positive rate).

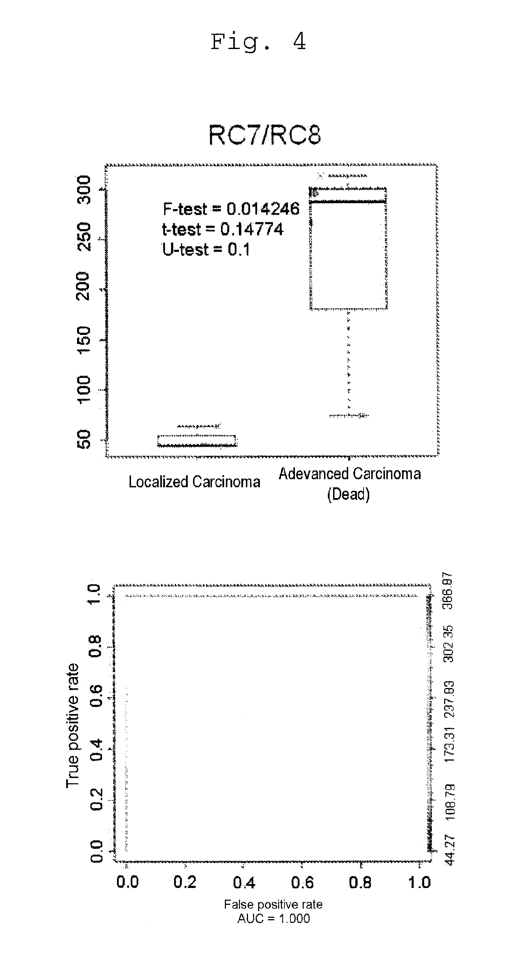

[Figure 4] The upper panel: a boxplot showing distribution of the ratio of expression level of RC7 to RC8 (RC7/RC8) in serum from renal cell carcinoma patients (localized and advanced carcinoma (dead case)). The lower panel: a ROC curve of the ratio of expression level of RC7 to RC8 (RC7/RC8) (vertical line: true positive rate; horizontal line: false positive rate).

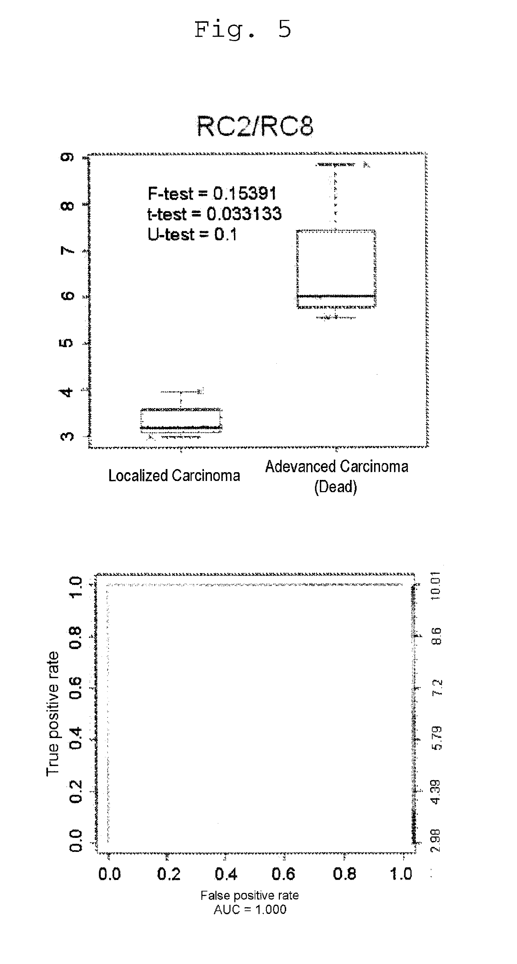

[Figure 5] The upper panel: a boxplot showing distribution of the ratio of expression level of RC2 to RC8 (RC2/RC8) in serum from renal cell carcinoma patients (localized and advanced carcinoma (dead case)). The lower panel: a ROC curve of the ratio of expression level of RC2 to RC8 (RC2/RC8) (vertical line: true positive rate; horizontal line: false positive rate).

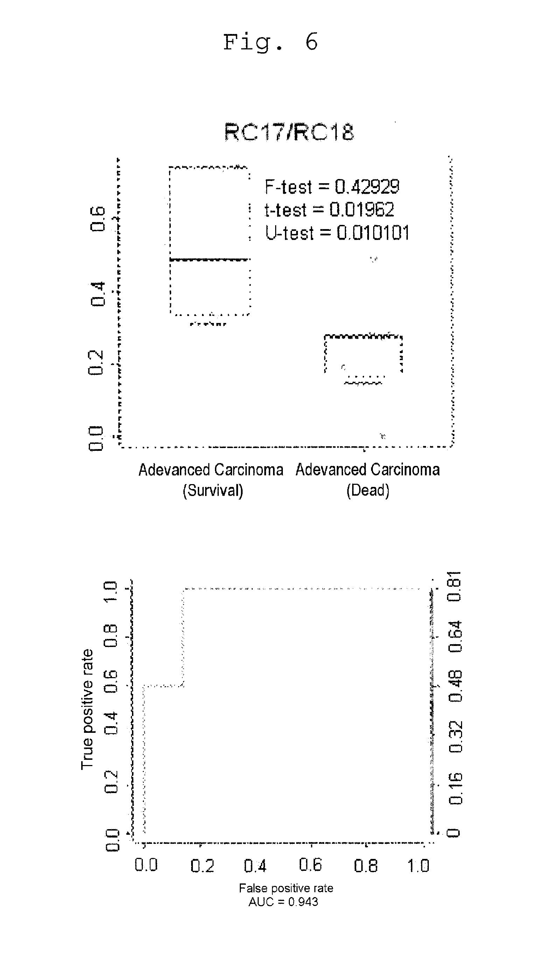

[Figure 6] The upper panel: a boxplot showing distribution of the ratio of expression level of RC17 to RC18 (RC17/RC18) in serum from renal cell carcinoma patients (advanced carcinoma (survival case) and advanced carcinoma (dead case)). The lower panel: a ROC curve of the ratio of expression level of RC17 to RC18 (RC17/RC18) (vertical line: true positive rate; horizontal line: false positive rate).

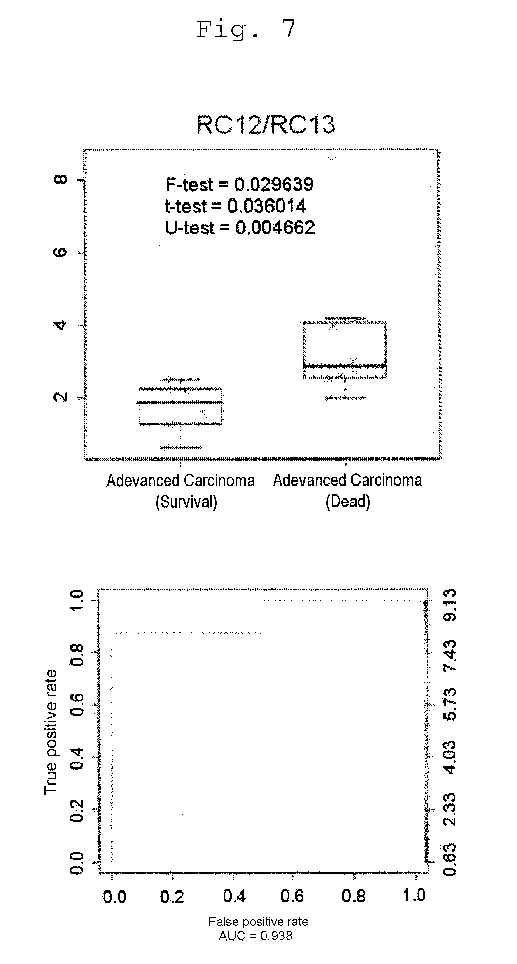

[Figure 7] The upper panel: a boxplot showing distribution of the ratio of expression level of RC12 to RC13 (RC12/RC13) in serum from renal cell carcinoma patients (advanced carcinoma (survival case) and advanced carcinoma (dead case)). The lower panel: a ROC curve of the ratio of expression level of RC12 to RC13 (RC12/RC13) (vertical line: true positive rate; horizontal line: false positive rate).

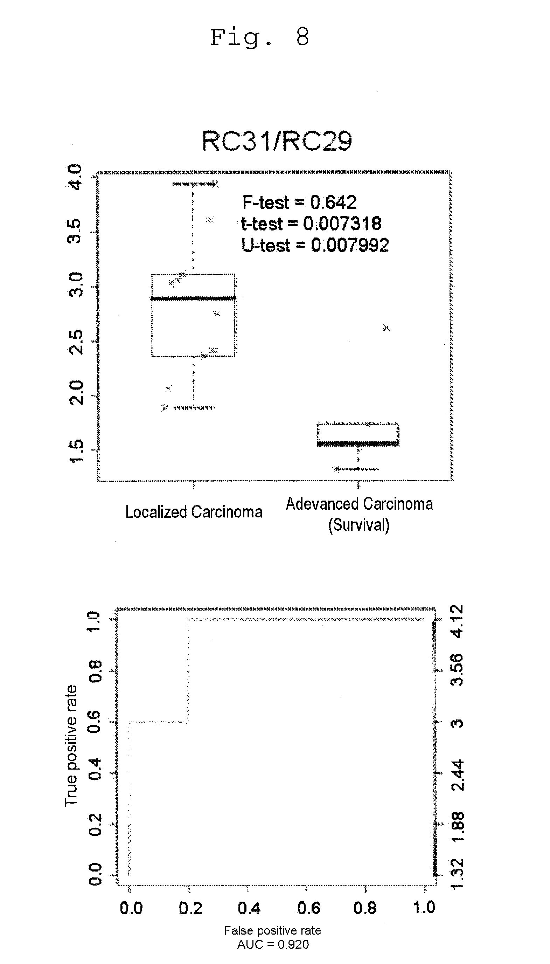

[Figure 8] The upper panel: a boxplot showing distribution of the ratio of expression level of RC31 to RC29 (RC31/RC29) in serum from renal cell carcinoma patients (localized and advanced carcinoma (survival case)). The lower panel: a ROC curve of the ratio of expression level of RC31 to RC29 (RC31/RC29) (vertical line: true positive rate; horizontal line: false positive rate).

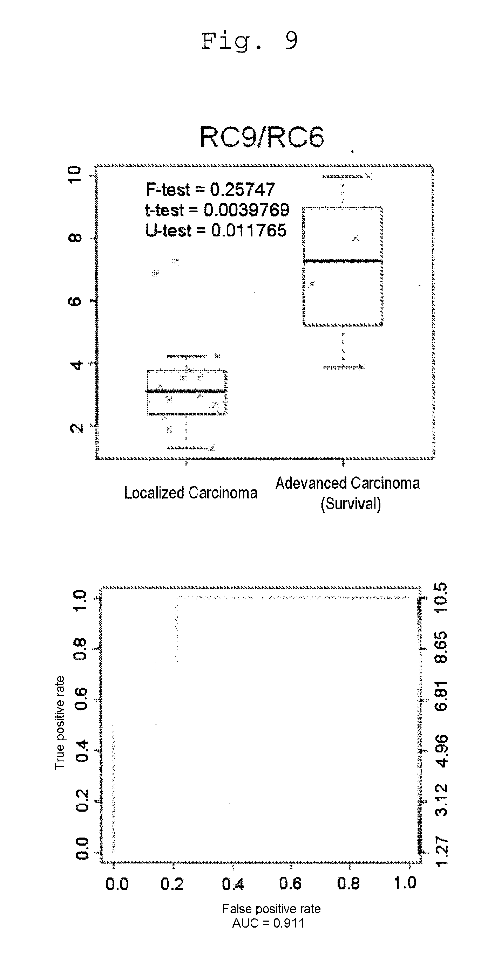

[Figure 9] The upper panel: a boxplot showing distribution of the ratio of expression level of RC9 to RC6 (RC9/RC6) in serum from renal cell carcinoma patients (localized and advanced carcinoma (survival case)). The lower panel: a ROC curve of the ratio of expression level of RC9 to RC6 (RC9/RC6) (vertical line: true positive rate; horizontal line: false positive rate).

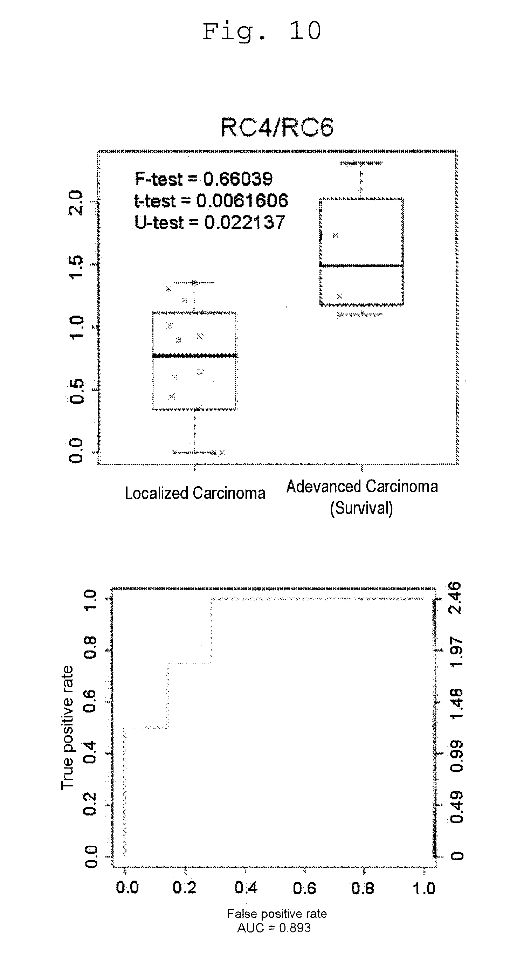

[Figure 10] The upper panel: a boxplot showing distribution of the ratio of expression level of RC4 to RC6 (RC4/RC6) in serum from renal cell carcinoma patients (localized and advanced carcinoma (survival case)). The lower panel: a ROC curve of the ratio of expression level of RC4 to RC6 (RC4/RC6) (vertical line: true positive rate; horizontal line: false positive rate).

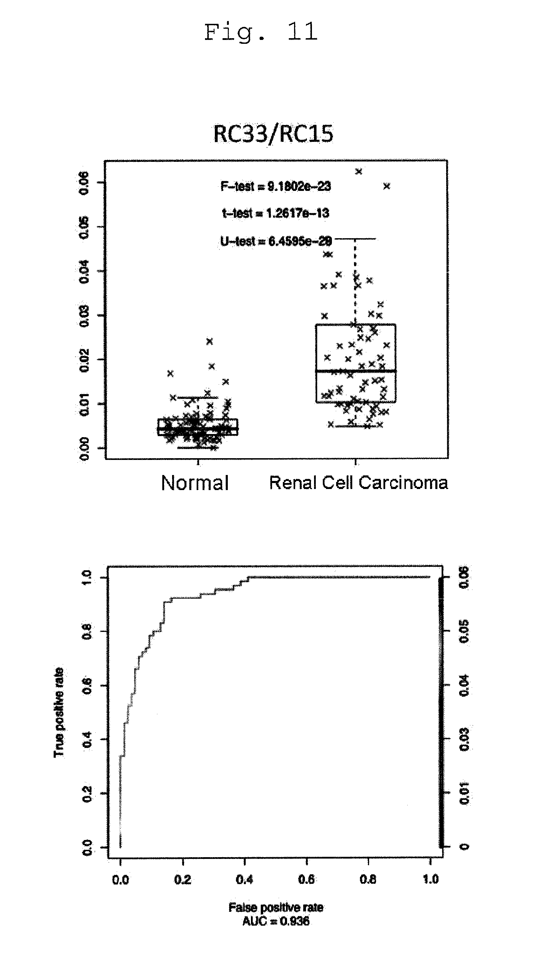

[Figure 11] The upper panel: a boxplot showing distribution of the ratio of expression level of RC33 to RC15 (RC33/RC15) in serum from normal subjects and renal cell carcinoma patients. The lower panel: a ROC curve of the ratio of expression level of RC33 to RC15 (RC33/RC15) (vertical line: true positive rate; horizontal line: false positive rate).

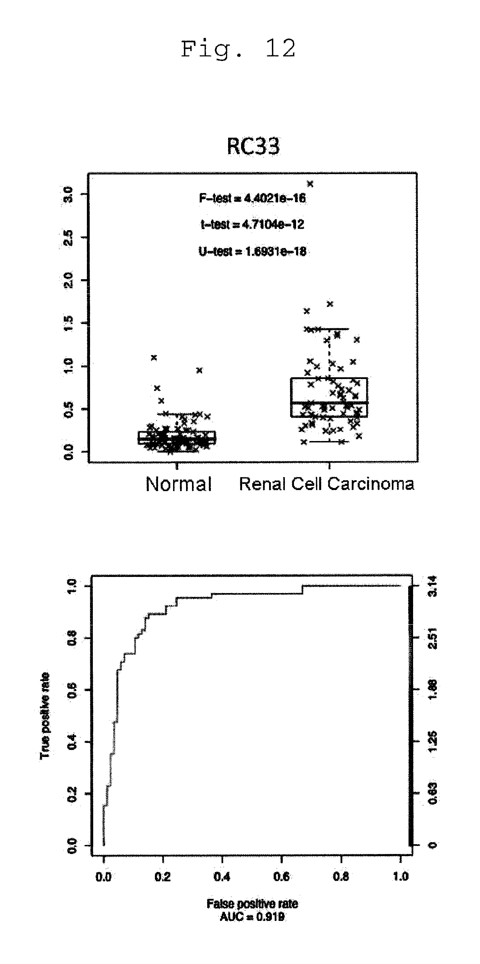

[Figure 12] The upper panel: a boxplot showing distribution of the expression level of RC33 in serum from normal subjects and renal cell carcinoma patients. The lower panel: a ROC curve of the expression level of RC33 (vertical line: true positive rate; horizontal line: false positive rate).

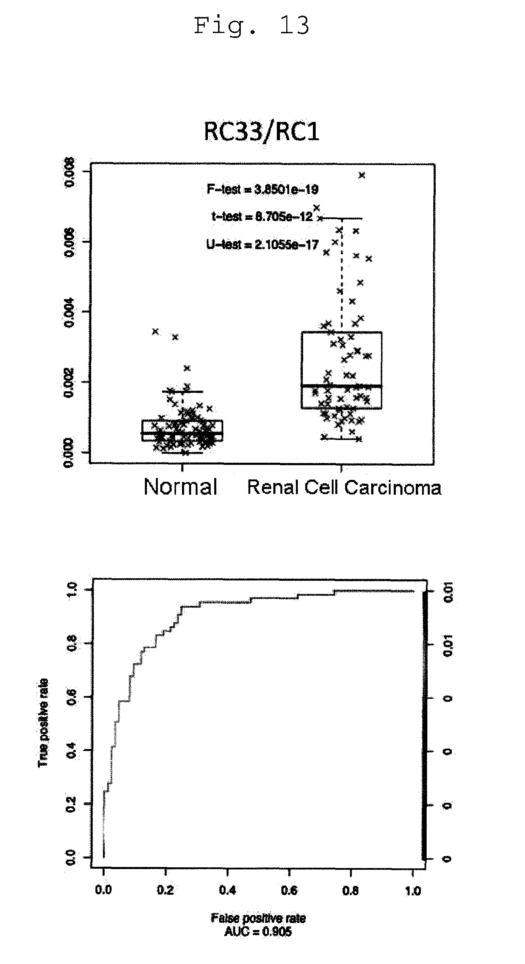

[Figure 13] The upper panel: a boxplot showing distribution of the ratio of expression level of RC33 to RC1 (RC33/RC1) in serum from normal subjects and renal cell carcinoma patients. The lower panel: a ROC curve of the ratio of expression level of RC33 to RC1 (RC33/RC1) (vertical line: true positive rate; horizontal line: false positive rate).

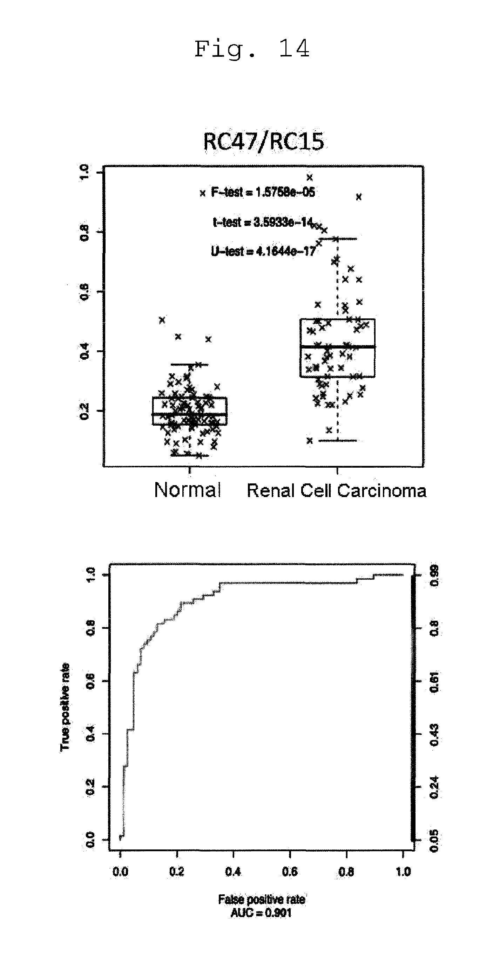

[Figure 14] The upper panel: a boxplot showing distribution of the ratio of expression level of RC47 to RC15 (RC47/RC15) in serum from normal subjects and renal cell carcinoma patients. The lower panel: a ROC curve of the ratio of expression level of RC47 to RC15 (RC47/RC15) (vertical line: true positive rate; horizontal line: false positive rate).

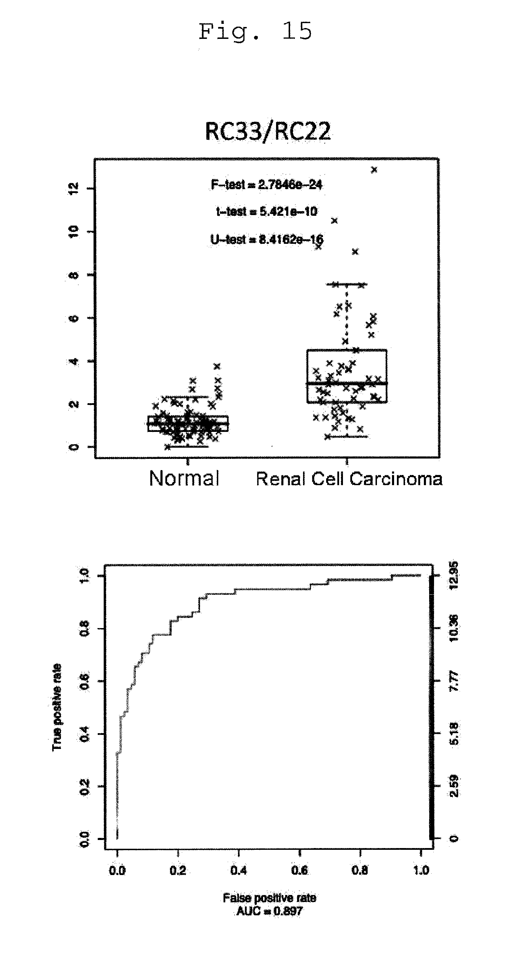

[Figure 15] The upper panel: a boxplot showing distribution of the ratio of expression level of RC33 to RC22 (RC33/RC22) in serum from normal subjects and renal cell carcinoma patients. The lower panel: a ROC curve of the ratio of expression level of RC33 to RC22 (RC33/RC22) (vertical line: true positive rate; horizontal line: false positive rate).

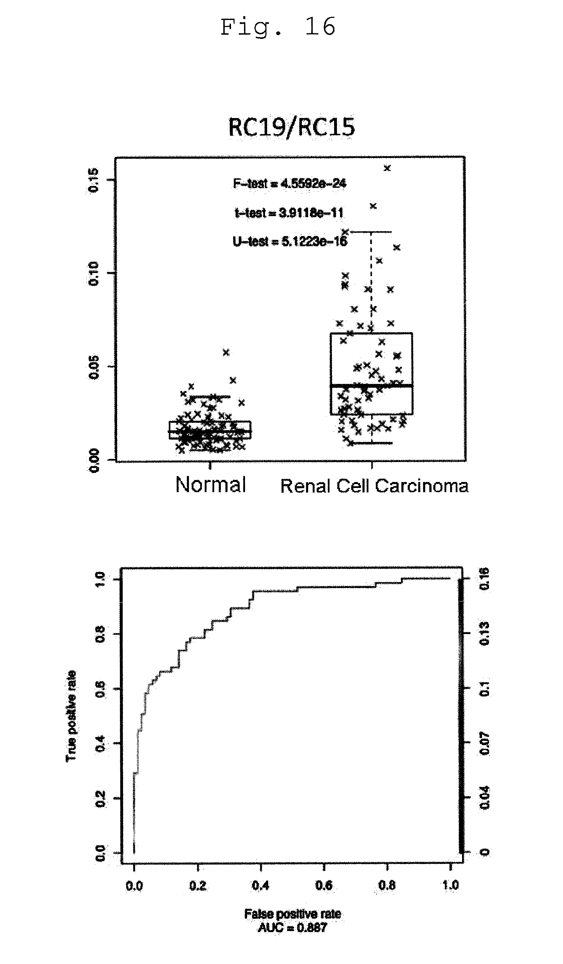

[Figure 16] The upper panel: a boxplot showing distribution of the ratio of expression level of RC19 to RC15 (RC19/RC15) in serum from normal subjects and renal cell carcinoma patients. The lower panel: a ROC curve of the ratio of expression level of RC19 to RC15 (RC19/RC15) (vertical line: true positive rate; horizontal line: false positive rate).

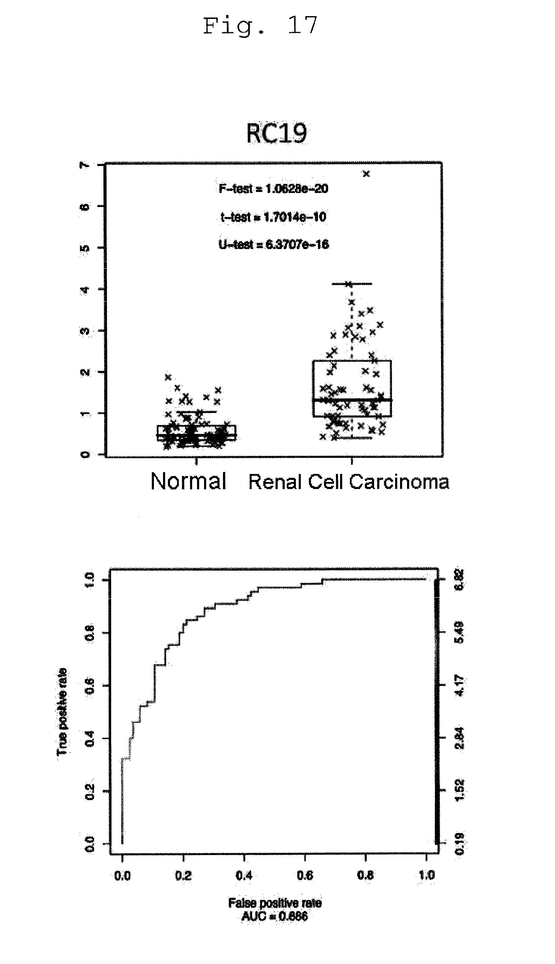

[Figure 17] The upper panel: a boxplot showing distribution of the expression level of RC19 in serum from normal subjects and renal cell carcinoma patients. The lower panel: a ROC curve of the expression level of RC19 (vertical line: true positive rate; horizontal line: false positive rate).

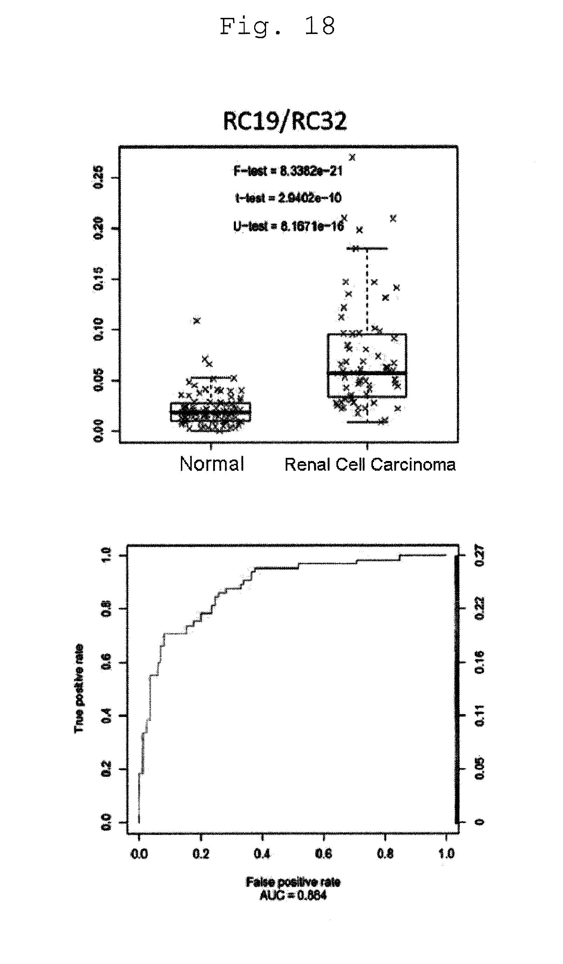

[Figure 18] The upper panel: a boxplot showing distribution of the ratio of expression level of RC19 to RC32 (RC19/RC32) in serum from normal subjects and renal cell carcinoma patients. The lower panel: a ROC curve of the ratio of expression level of RC19 to RC32 (RC19/RC32) (vertical line: true positive rate; horizontal line: false positive rate).

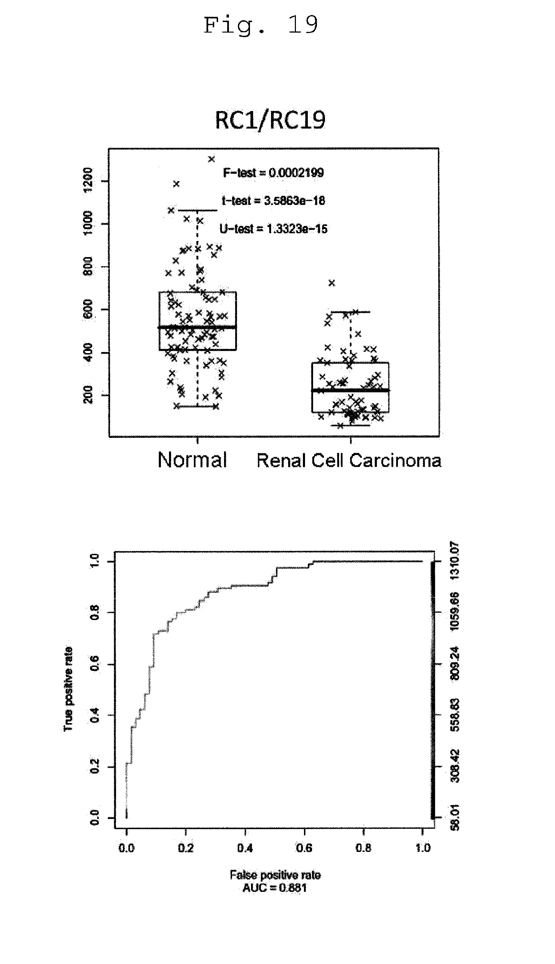

[Figure 19] The upper panel: a boxplot showing distribution of the ratio of expression level of RC1 to RC19 (RC1/RC19) in serum from normal subjects and renal cell carcinoma patients. The lower panel: a ROC curve of the ratio of expression level of RC1 to RC19 (RC1/RC19) (vertical line: true positive rate; horizontal line: false positive rate).

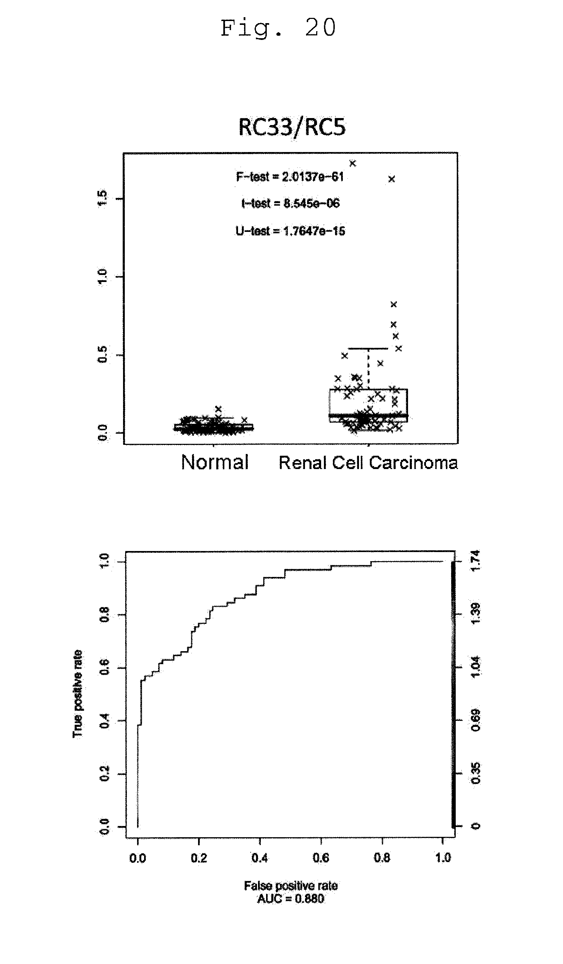

[Figure 20] The upper panel: a boxplot showing distribution of the ratio of expression level of RC33 to RC5 (RC33/RC5) in serum from normal subjects and renal cell carcinoma patients. The lower panel: a ROC curve of the ratio of expression level of RC33 to RC5 (RC33/RC5) (vertical line: true positive rate; horizontal line: false positive rate).

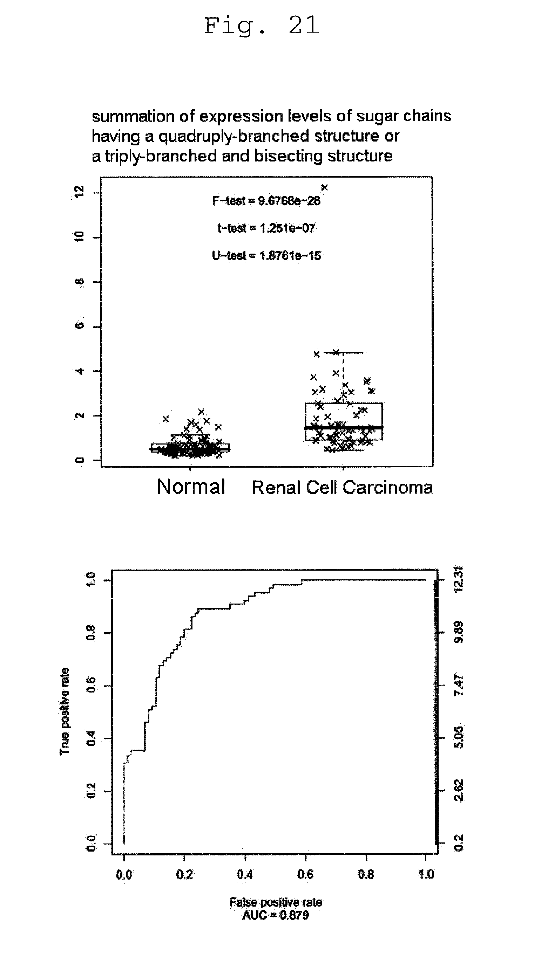

[Figure 21] The upper panel: a boxplot showing distribution of the summation of expression levels of sugar chains having a tetra-antennary structure or a tri-antennary and bisecting structure in serum from normal subjects and renal cell carcinoma patients. The lower panel: a ROC curve of the summation of expression levels of sugar chains having a tetra-antennary structure or a tri-antennary and bisecting structure (vertical line: true positive rate; horizontal line: false positive rate).

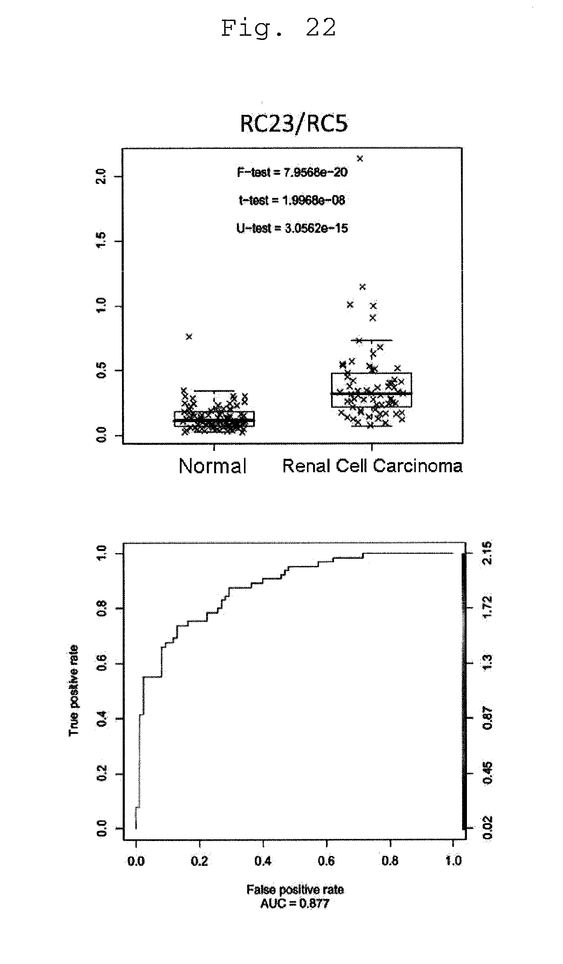

[Figure 22] The upper panel: a boxplot showing distribution of the ratio of expression level of RC23 to RC5 (RC23/RC5) in serum from normal subjects and renal cell carcinoma patients. The lower panel: a ROC curve of the ratio of expression level of RC23 to RC5 (RC23/RC5) (vertical line: true positive rate; horizontal line: false positive rate).

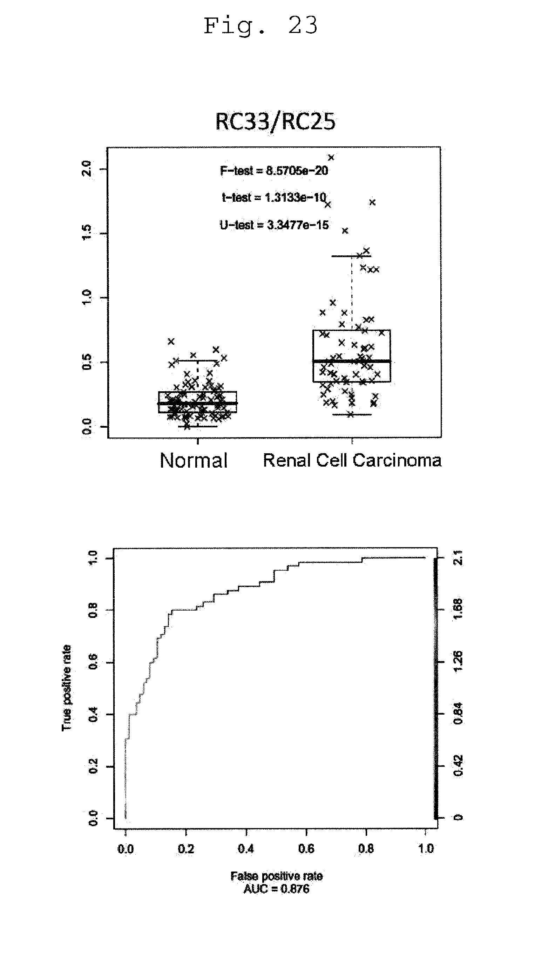

[Figure 23] The upper panel: a boxplot showing distribution of the ratio of expression level of RC33 to RC25 (RC33/RC25) in serum from normal subjects and renal cell carcinoma patients. The lower panel: a ROC curve of the ratio of expression level of RC33 to RC25 (RC33/RC25) (vertical line: true positive rate; horizontal line: false positive rate).

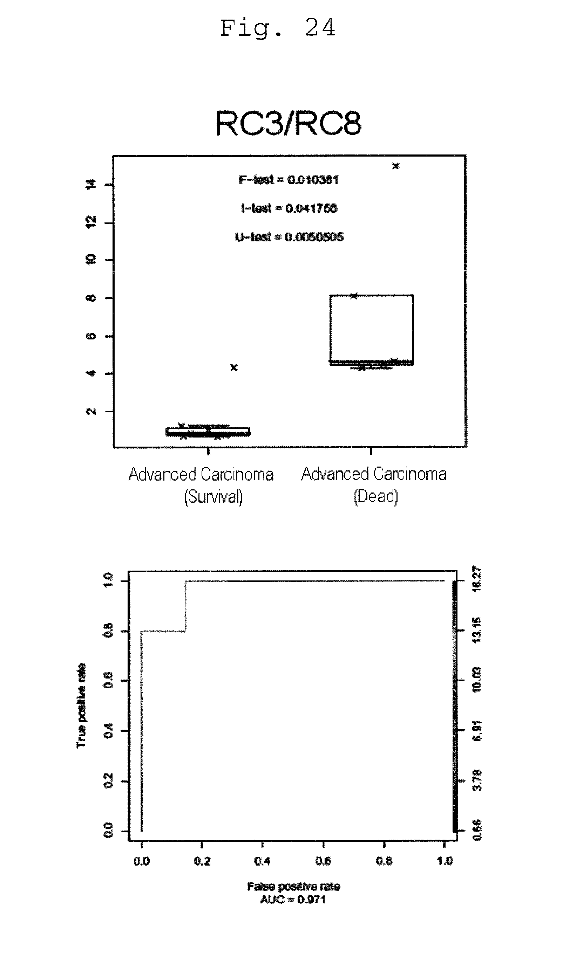

[Figure 24] The upper panel: a boxplot showing distribution of the ratio of expression level of RC3 to RC8 (RC3/RC8) in serum from renal cell carcinoma patients (advanced carcinoma (survival case) and advanced carcinoma (dead case)). The lower panel: a ROC curve of the ratio of expression level of RC3 to RC8 (RC3/RC8) (vertical line: true positive rate; horizontal line: false positive rate).

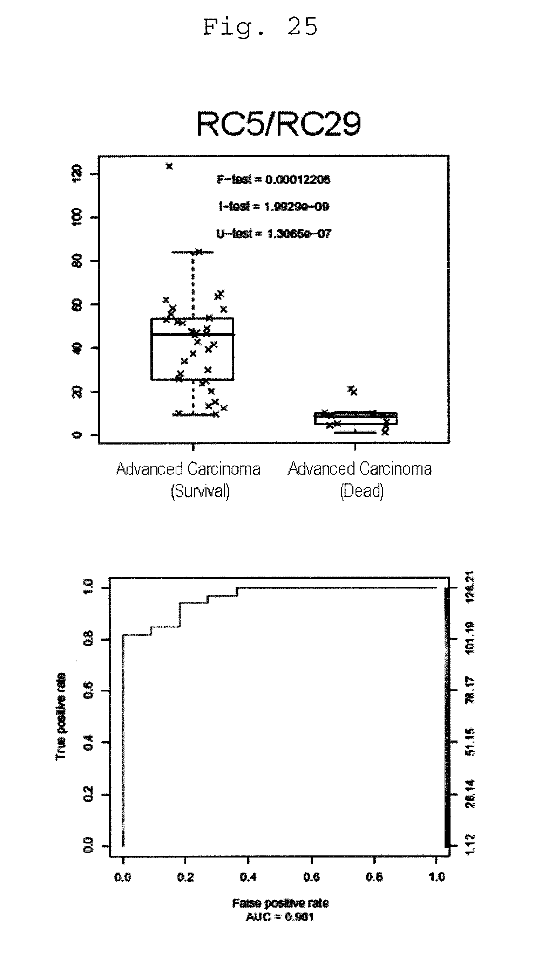

[Figure 25] The upper panel: a boxplot showing distribution of the ratio of expression level of RC5 to RC29 (RC5/RC29) in serum from renal cell carcinoma patients (advanced carcinoma (survival case) and advanced carcinoma (dead case)). The lower panel: a ROC curve of the ratio of expression level of RC5 to RC29 (RC5/RC29) (vertical line: true positive rate; horizontal line: false positive rate).

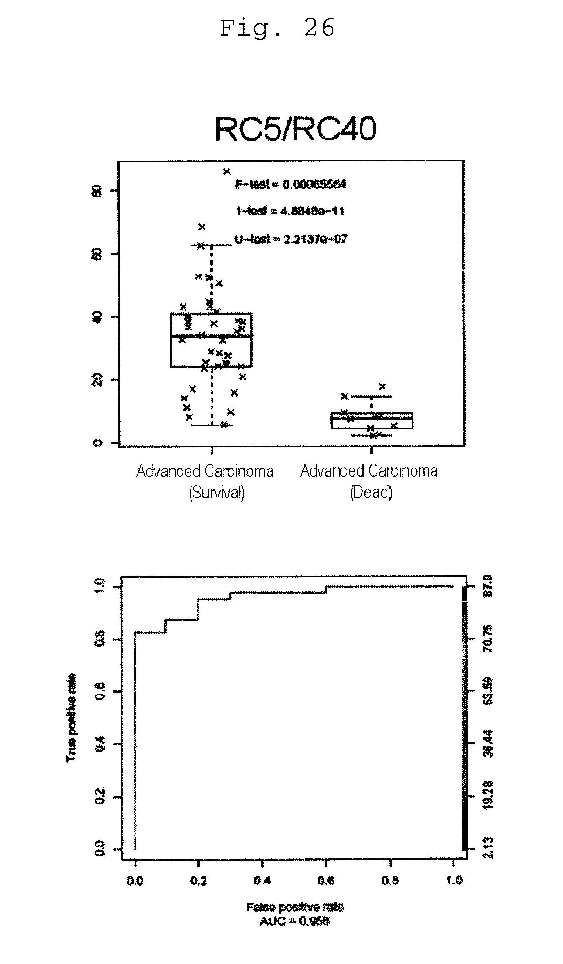

[Figure 26] The upper panel: a boxplot showing distribution of the ratio of expression level of RC5 to RC40 (RC5/RC40) in serum from renal cell carcinoma patients (advanced carcinoma (survival case) and advanced carcinoma (dead case)). The lower panel: a ROC curve of the ratio of expression level of RC5 to RC40 (RC5/RC40) (vertical line: true positive rate; horizontal line: false positive rate).

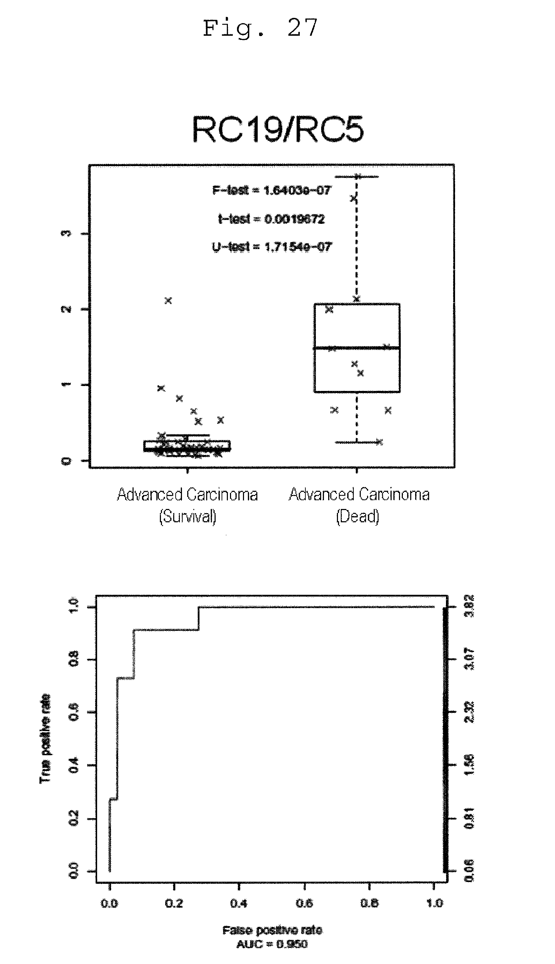

[Figure 27] The upper panel: a boxplot showing distribution of the ratio of expression level of RC19 to RC5 (RC19/RC5) in serum from renal cell carcinoma patients (advanced carcinoma (survival case) and advanced carcinoma (dead case)). The lower panel: a ROC curve of the ratio of expression level of RC19 to RC5 (RC19/RC5) (vertical line: true positive rate; horizontal line: false positive rate).

### BEST MODE FOR CARRYING OUT THE INVENTION

According to the present invention, all N-linked sugar chains in serum of a test subject are collected using glycoblotting method and subjected to MALDI-TOF mass spectrometry to obtain a quantitive profile. In this regard, known amount of an oligosaccharide may be added as an internal standard so that the abundance (herein also referred to as "the expression level") of detected N-linked sugar chain in blood is calculated readily from the area above the spectrum. Sugar chain analysis may be conducted according to the methods as described in Nishimura et al., supra. (Angew. Chem. Int. Ed., 44, 91-96 (2005)), Miura et al. (Miura Y., Hato M., Shinohara Y., Kuramoto H., Furukawa J.-I., Kurogochi M., Shimaoka H., Tada M., Nakanishi K., Ozaki M., Todo S., and Nishimura S.-I.), "BlotGlycoABC™, an Integrated Glycoblotting Technique for Rapid and Large Scale Clinical Glycomics" Molecular & Cellular Proteomics 7:370-377 (2008), Furukawa et al. (Furukawa J.-I., Shinohara Y., Kuramoto H., Miura Y., Shimaoka H., Kurogochi M., Nakano M., Nishimura S.-I.), "Comprehensive Approach to Structural and Functional Glycomics Based on Chemoselective Glycoblotting and Sequential Tag Conversion" and WO2009/044900.

Then, an expression level of one sugar chain as detected, a ratio of expression level of one sugar chain to another sugar chain as detected or a summation of expression levels of sugar chains as detected is calculated, and the presence of renal cell carcinoma or the degree of malignancy of renal cell carcinoma in the patient is determined utilizing said expression level, said ratio of expression level or said summation of expression levels as an index of the determination.

For example, the detectable N-linked sugar chains that may be used in the method of the invention include, but not limited to, those as shown in the following Table 1.

**[Table 1]**

| Sugar Chain | composition |
|---|---|
| RC1 | (Hex)2(HexNAc)2(NeuAc)2 + (Man)3(GlcNAc)2 |
| RC2 | (Hex)1(HexNAc)3(dHex)1(NeuAc)1 + (Man)3(GlcNAc)2 |
| RC3 | (Hex)3(HexNAc)3(dHex)1(NeuAc)2 + (Man)3(GlcNAc)2 |
| RC4 | (Hex)3(HexNAc)3(dHex)1(NeuAc)1 + (Man)3(GlcNAc)2 |
| RC5 | (Hex)2(HexNAc)2(dHex)1 + (Man)3(GlcNAc)2 |
| RC6 | (Hex)2(HexNAc)3 + (Man)3(GlcNAc)2 |
| RC7 | (Hex)3(HexNAc)3(NeuAc)3 + (Man)3(GlcNAc)2 |
| RC8 | (Hex)1(HexNAc)1(dHex)1(NeuAc)1 + (Man)3(GlcNAc)2 |
| RC9 | (Hex)1(HexNAc)3(NeuAc)1 + (Man)3(GlcNAc)2 |
| RC12 | (Hex)2(HexNAc)3(dHex)1 + (Man)3(GlcNAc)2 |
| RC13 | (Hex)2(HexNAc)2 + (Man)3(GlcNAc)2 |
| RC15 | (Hex)2(HexNAc)2(NeuAc)1+ (Man)3(GlcNAc)2 |
| RC17 | (Hex)1(HexNAc)2 + (Man)3(GlcNAc)2 |
| RC18 | (Hex)3 + (Man)3(GlcNAc)2 |
| RC19 | (Hex)3(HexNAc)4 + (Man)3(GlcNAc)2 |
| RC22 | (Hex)4 + (Man)3(GlcNAc)2 |
| RC23 | (Hex)1(HexNAc)3(dHex)1 + (Man)3(GlcNAc)2 |
| RC25 | (Hex)2 + (Man)3(GlcNAc)2 |
| RC29 | (Hex)1(HexNAc)1(NeuAc)1 + (Man)3(GlcNAc)2 |
| RC31 | (Hex)6 + (Man)3(GlcNAc)2 |
| RC32 | (Hex)2(HexNAc)2(dHex)1(NeuAc)1 + (Man)3(GlcNAc)2 |
| RC33 | (Hex)3(HexNAc)3(NeuAc)1 + (Man)3(GlcNAc)2 |
| RC40 | (Hex)2(HexNAc)1(NeuAc)1 + (Man)3(GlcNAc)2 |
| RC47 | (Hex)2(HexNAc)2(dHex)1(NeuAc)2 + (Man)3(GlcNAc)2 |

In Table 1, "Man" represents mannose, "GlcNAc" represents N-acetylglucosamine, "Hex" represents hexose, "dHex" represents deoxyhexose, "HexNAc" represents N-acetylhexosamine, and "NeuAc" represents N-acetylneuraminic acid.

The detectable N-linked sugar chains that may be used in the method of the invention also include sugar chains having a tetra-antennary structure or a tri-antennary and bisecting structure. The term "tetra-antennary structure" in "sugar chain having a tetra-antennary structure or a tri-antennary and bisecting structure" means a structure wherein a monnose, which is α-(1→6)-linked to the core sugar chain, branches into biantennary sugar chains with β-(1→6)- and β-(1→2)-liked N-acetylglucosamine residues and also another monnose, which is α-(1→3)-linked to the core sugar chain, branches into biantennary sugar chains with β-(1→4)- and β-(1→2)-liked N-acetylglucosamine residues. The term "tri-antennary and bisecting structure" means a structure which lacks one of the four branched chains of the tetra-antennary structure as mentioned above but has a β-(1→4)-liked N-acetylglucosamine residue at the terminus of the core sugar chain (i.e., "bisecting GlcNAc"). For example, these sugar chains include, but not limited to, those having a structure as follows.
Examples of sugar chain having a tetra-antennary structure: 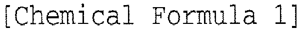 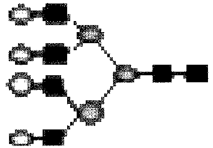 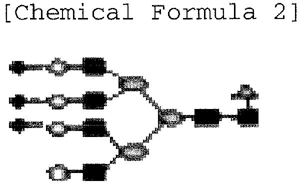 Examples of sugar chain having a tri-antennary and bisecting structure: 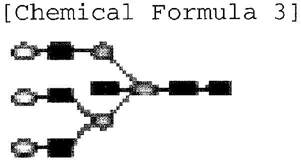 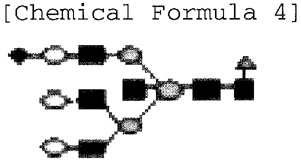 In the above formulae,
■ represents N-acetylglucsamine (GlcNAc),
▲ represents fucose (Fuc), ● represents mannose (Man),
○ represents galactose (Gal), ● represents glucose (Glc),
◆ represents N-acetylsialic acid (NeuAc).

In the present invention, it has been found that an expression level of one sugar chain as detected by the glycoblotting method described above, a ratio of expression level of one sugar chain to another sugar chain as detected by the glycoblotting method described above, and a summation of expression levels of sugar chains as detected by the glycoblotting method described above correlate with the presence of renal cell carcinoma. For example, among the sugar chains exemplified above, RC1, RC5, RC15, RC19, RC22, RC23, RC25, RC32, RC33, RC47 and sugar chains having a tetra-antennary structure or a tri-antennary and bisecting structure are found to correlate with the presence of renal cell carcinoma.

Specifically, an expression level of RC33 and RC19 has been found to increase in renal cell carcinoma.
Also, following ratios of expression level of the sugar chains:
RC33 to RC15 (RC33/RC15),
RC33 to RC1 (RC33/RC1),
RC47 to RC15 (RC47/RC15),
RC33 to RC22 (RC33/RC22),
RC19 to RC15 (RC19/RC15),
RC19 to RC32 (RC19/RC32),
RC33 to RC5 (RC33/RC5),
RC23 to RC5 (RC23/RC5) and
RC33 to RC25 (RC33/RC25)
have been found to increase in renal cell carcinoma, and a ratio of RC1 to RC19 (RC1/RC19) has been found to decreases in renal cell carcinoma.
Additionally, a summation of expression levels of sugar chains having a tetra-antennary structure or a tri-antennary and bisecting structure has been found to increase in renal cell carcinoma.

Furthermore, it has been found that a ratio of expression level of one sugar chain to another sugar chain, as detected by the glycoblotting method described above, correlates with the degree of malignancy of renal cell carcinoma. For example, among the sugar chains exemplified above, RC2, RC3, RC4, RC5, RC6, RC7, RC8, RC9, RC12, RC13, RC17, RC18, RC19, RC29, RC31 and RC40 are found to correlate with the degree of malignancy of renal cell carcinoma.

Specifically, following ratios of expression level of the sugar chains:
RC9 to RC6 (RC9/RC6) and
RC4 to RC6 (RC4/RC6)
have been found to increase in advanced renal cell carcinoma, and a ratio of expression level of RC31 to RC29 (RC31/RC29) has been found to decrease in advanced renal cell carcinoma.

Additionally, following ratios of expression level of the sugar chains:
RC3 to RC6 (RC3/RC6),
RC7 to RC8 (RC7/RC8),
RC2 to RC8 (RC2/RC8),
RC12 to RC13 (RC12/RC13),
RC3 to RC8 (RC3/RC8) and
RC19 to RC5 (RC19/RC5)
   have been found to increase in poor-prognosis advanced renal cell carcinoma, and
   following ratios of expression level of the sugar chains:
RC17 to RC18 (RC17/RC18),
RC5 to RC29 (RC5/RC29) and
RC5 to RC40 (RC5/RC40)
have been found to decrease in poor-prognosis advanced renal cell carcinoma.

Thus, the presence and the degree of malignancy of renal cell carcinoma can be determined by conducting a comprehensive sugar chain analysis of N-linked sugar chains in serum using the glycoblotting method as described above and utilizing an expression level of a sugar chain or a ratio of expression level of sugar chains as a marker for such determination (see the Examples provided below).
Among others, expression levels of RC33 and RC19, ratios of expression level RC33/RC15, RC33/RC1, RC47/RC15, RC33/RC22, RC19/RC15, RC19/RC32, RC33/RC5, RC23/RC5, RC33/RC25 and RC1/RC19 and a summation of expression levels of sugar chains having a tetra-antennary structure or a tri-antennary and bisecting structure show a significant difference between normal subjects and renal cell carcinoma patients. Therefore, these are useful as a measure to determine the presence of renal cell carcinoma.
Also, ratios of expression level RC31/RC29, RC9/RC6 and RC4/RC6 show a significant difference between localized carcinoma and advanced carcinoma. Therefore, these are useful as a measure for diagnosis of advanced renal cell carcinoma. Furthermore, ratios of expression level RC3/RC6, RC7/RC8, RC2/RC8, RC17/RC18, RC12/RC13, RC3/RC8, RC5/RC29, RC5/RC40 and RC19/RC5 show a significant difference between survival case and dead case of renal cell carcinoma. Therefore, these are useful as a predictive marker for prognosis to identify renal cell carcinoma with a risk of dying.
Additionally, such ratios of expression level of sugar chains may be used in combination to improve diagnostic efficiency.

The following examples are provided to illustrate the invention and are in no way intended to limit the scope of the invention.

### Example 1

Using the serum samples from 31 renal cell carcinoma patients (17 cases of localized carcinoma, 6 cases of advanced carcinoma (survival) and 8 cases of advanced carcinoma (dead)), a comprehensive analysis of N-linked sugar chains was conducted according to the method of Nishimura et al., supra. The N-linked sugar chains in serum are captured and purified, and a quantitative profile thereof was obtained by MALDI-TOF MS. Figure 1 shows a MALDI spectrum of N-linked sugar chains in renal cell carcinoma patients. The expression level of each sugar chain was calculated from the area ratio on the spectrum between the sugar chain and the known amount of an oligosaccharide added as an internal standard. Among the sugar chains as detected, we focused on the sugar chains RC3 and RC6 and calculated a ratio of the expression level thereof (RC3/RC6). The obtained value of the ratio was significantly high in advanced carcinoma (dead case) (AUC=1.000, Figure 2 and Figure 3). Thus, the ratio of expression level RC3/RC6 increases with the worsening of renal cell carcinoma, and it is available as a predictive marker for prognosis to identify advanced renal cell carcinoma with a risk of dying.

### Example 2

Among the sugar chains detected in Example 1, we focused on the sugar chains RC7 and RC8 and calculated a ratio of the expression level thereof (RC7/RC8). The obtained value of the ratio was significantly higher in advanced carcinoma (dead case) than localized carcinoma (AUC=1.000, Figure 4). Thus, the ratio of expression level RC7/RC8 is available as a predictive marker for prognosis to identify advanced renal cell carcinoma with a risk of dying.

### Example 3

Among the sugar chains detected in Example 1, we focused on the sugar chains RC2 and RC8 and calculated the ratio of the expression level thereof (RC2/RC8). The obtained value of the ratio was significantly higher in advanced carcinoma (dead case) than localized carcinoma (AUC=1.000, Figure 5). Thus, the ratio of expression level RC2/RC8 is available as a predictive marker for prognosis to identify advanced renal cell carcinoma with a risk of dying.

### Example 4

Among the sugar chains detected in Example 1, we focused on the sugar chains RC17 and RC18 and calculated the ratio of the expression level thereof (RC17/RC18). The obtained value of the ratio was significantly lower in advanced carcinoma (dead case) than advanced carcinoma (survival case) (AUC=0.943, Figure 6). Thus, the ratio of expression level RC17/RC18 is available as a predictive marker for prognosis to identify advanced renal cell carcinoma with a risk of dying.

### Example 5

Among the sugar chains detected in Example 1, we focused on the sugar chains RC12 and RC13 and calculated the ratio of the expression level thereof (RC12/RC13). The obtained value of the ratio was significantly higher in advanced carcinoma (dead case) than advanced carcinoma (survival case) (AUC=0.938, Figure 7). Thus, the ratio of expression level RC12/RC13 is available as a predictive marker for prognosis to identify advanced renal cell carcinoma with a risk of dying.

### Example 6

Among the sugar chains detected in Example 1, we focused on the sugar chains RC31 and RC29 and calculated the ratio of the expression level thereof (RC31/RC29). The obtained value of the ratio was significantly higher in advanced carcinoma (survival case) than localized carcinoma (AUC=0.920, Figure 8). Thus, the ratio of expression level RC31/RC29 is available as a diagnostic marker to distinguish between localized carcinoma and advanced carcinoma.

### Example 7

Among the sugar chains detected in Example 1, we focused on the sugar chains RC9 and RC6 and calculated the ratio of the expression level thereof (RC9/RC6). The obtained value of the ratio was significantly lower in advanced carcinoma (survival case) than localized carcinoma (AUC=0.911, Figure 9). Thus, the ratio of expression level RC9/RC6 is available as a diagnostic marker to distinguish between localized carcinoma and advanced carcinoma.

### Example 8

Among the sugar chains detected in Example 1, we focused on the sugar chains RC4 and RC6 and calculated the ratio of the expression level thereof (RC4/RC6). The obtained value of the ratio was significantly lower in advanced carcinoma (survival case) than localized carcinoma (AUC=0.893, Figure 10). Thus, the ratio of expression level RC4/RC6 is available as a diagnostic marker to distinguish between localized carcinoma and advanced carcinoma.

### Example 9

Using the serum samples from 65 renal cell carcinoma patients (between 30s and 90s) and 85 healthy individuals (between 40s and 70s), similar analysis was conducted as described in Example 1.
Among the sugar chains as detected, we focused on the sugar chains RC33 and RC15 and calculated a ratio of the expression level thereof (RC33/RC15). The obtained value of the ratio was significantly high in renal cell carcinoma (AUC=0.936, Figure 11). Thus, the ratio of expression level RC33/RC15 increases in renal cell carcinoma patients in a selective manner, and it is available as a diagnostic marker to identify renal cell carcinoma.

### Example 10

Among the sugar chains detected in Example 9, we focused on the sugar chains RC33 and found that the expression level was significantly higher in renal cell carcinoma patients than healthy individuals (AUC=0.919, Figure 12). Thus, the expression level of RC33 is available as a diagnostic marker to identify renal cell carcinoma.

### Example 11

Among the sugar chains detected in Example 9, we focused on the sugar chains RC33 and RC1 and calculated the ratio of the expression level thereof (RC33/RC1). The obtained value of the ratio was significantly high in renal cell carcinoma (AUC=0.905, Figure 13). Thus, the value of the ratio of expression level RC33/RC1 increases in renal cell carcinoma patients in a selective manner, and it is available as a diagnostic marker to identify renal cell carcinoma.

### Example 12

Among the sugar chains detected in Example 9, we focused on the sugar chains RC47 and RC15 and calculated the ratio of the expression level thereof (RC47/RC15). The obtained value of the ratio was significantly high in renal cell carcinoma (AUC=0.901, Figure 14). Thus, the value of the ratio of expression level RC47/RC15 increases in renal cell carcinoma patients in a selective manner, and it is available as a diagnostic marker to identify renal cell carcinoma.

### Example 13

Among the sugar chains detected in Example 9, we focused on the sugar chains RC33 and RC22 and calculated the ratio of the expression level thereof (RC33/RC22). The obtained value of the ratio was significantly high in renal cell carcinoma (AUC=0.897, Figure 15). Thus, the value of the ratio of expression level RC33/RC22 increases in renal cell carcinoma patients in a selective manner, and it is available as a diagnostic marker to identify renal cell carcinoma.

### Example 14

Among the sugar chains detected in Example 9, we focused on the sugar chains RC19 and RC15 and calculated the ratio of the expression level thereof (RC19/RC15). The obtained value of the ratio was significantly high in renal cell carcinoma (AUC=0.887, Figure 16). Thus, the value of the ratio of expression level RC19/RC15 increases in renal cell carcinoma patients in a selective manner, and it is available as a diagnostic marker to identify renal cell carcinoma.

### Example 15

Among the sugar chains detected in Example 9, we focused on the sugar chains RC19 and found that the expression level thereof was significantly higher in renal cell carcinoma patients than healthy individuals (AUC=0.886, Figure 17). Thus, the expression level of RC19 is available as a diagnostic marker to identify renal cell carcinoma.

### Example 16

Among the sugar chains detected in Example 9, we focused on the sugar chains RC19 and RC32 and calculated the ratio of the expression level thereof (RC19/RC32). The obtained value of the ratio was significantly high in renal cell carcinoma (AUC=0.884, Figure 18). Thus, the value of the ratio of expression level RC19/RC32 increases in renal cell carcinoma patients in a selective manner, and it is available as a diagnostic marker to identify renal cell carcinoma.

### Example 17

Among the sugar chains detected in Example 9, we focused on the sugar chains RC1 and RC19 and calculated the ratio of the expression level thereof (RC1/RC19). The obtained value of the ratio was significantly low in renal cell carcinoma (AUC=0.881, Figure 19). Thus, the value of the ratio of expression level RC1/RC19 decreases in renal cell carcinoma patients in a selective manner, and it is available as a diagnostic marker to identify renal cell carcinoma.

### Example 18

Among the sugar chains detected in Example 9, we focused on the sugar chains RC33 and RC5 and calculated the ratio of the expression level thereof (RC33/RC5). The obtained value of the ratio was significantly high in renal cell carcinoma (AUC=0.880, Figure 20). Thus, the value of the ratio of expression level RC33/RC5 increases in renal cell carcinoma patients in a selective manner, and it is available as a diagnostic marker to identify renal cell carcinoma.

### Example 19

Among the sugar chains detected in Example 9, we focused on the expression levels of sugar chains having a tetra-antennary structure or a tri-antennary and bisecting structure and calculated the summation thereof. The obtained value of the summation was significantly higher in renal cell carcinoma patients than healthy individuals (AUC=0.879, Figure 21). Thus, the summation of the expression levels of sugar chains having a tetra-antennary structure or a tri-antennary and bisecting structure is available as a diagnostic marker to identify renal cell carcinoma.

### Example 20

Among the sugar chains detected in Example 9, we focused on the sugar chains RC23 and RC5 and calculated the ratio of the expression level thereof (RC23/RC5). The obtained value of the ratio was significantly high in renal cell carcinoma (AUC=0.877, Figure 22). Thus, the value of the ratio of expression level RC23/RC5 increases in renal cell carcinoma patients in a selective manner, and it is available as a diagnostic marker to identify renal cell carcinoma.

### Example 21

Among the sugar chains detected in Example 9, we focused on the sugar chains RC33 and RC25 and calculated the ratio of the expression level thereof (RC33/RC25). The obtained value of the ratio was significantly high in renal cell carcinoma (AUC=0.876, Figure 23). Thus, the value of the ratio of expression level RC33/RC25 increases in renal cell carcinoma patients in a selective manner, and it is available as a diagnostic marker to identify renal cell carcinoma.

### Example 22

Using the serum samples from 65 renal cell carcinoma patients (40 cases of localized carcinoma, 14 cases of advanced carcinoma (survival) and 11 cases of advanced carcinoma (dead)), similar analysis was conducted as described in Example 1.
Among the sugar chains as detected, we focused on the sugar chains RC3 and RC8 and calculated the ratio of the expression level thereof (RC3/RC8). The obtained value of the ratio was significantly high in advanced carcinoma (dead case) (AUC=0.971, Figure 24). Thus, the ratio of expression level RC3/RC8 is available as a predictive marker for prognosis to identify advanced renal cell carcinoma with a risk of dying.

### Example 23

Among the sugar chains detected in Example 22, we focused on the sugar chains RC5 and RC29 and calculated the ratio of the expression level thereof (RC5/RC29). The obtained value of the ratio was significantly low in advanced carcinoma (dead case) (AUC=0.961, Figure 25). Thus, the value of the ratio of expression level RC5/RC29 is available as a predictive marker for prognosis to identify advanced renal cell carcinoma with a risk of dying.

### Example 24

Among the sugar chains detected in Example 22, we focused on the sugar chains RC5 and RC40 and calculated the ratio of the expression level thereof (RC5/RC40). The obtained value of the ratio was significantly low in advanced carcinoma (dead case) (AUC=0.958, Figure 26). Thus, the value of the ratio of expression level RC5/RC40 is available as a predictive marker for prognosis to identify advanced renal cell carcinoma with a risk of dying.

### Example 25

Among the sugar chains detected in Example 22, we focused on the sugar chains RC19 and RC5 and calculated the ratio of the expression level thereof (RC19/RC5). The obtained value of the ratio was significantly high in advanced carcinoma (dead case) (AUC=0.950, Figure 27). Thus, the value of the ratio of expression level RC19/RC5 is available as a predictive marker for prognosis to identify advanced renal cell carcinoma with a risk of dying.

### INDUSTRIAL APPLICABILITY

The method of the invention can be used in a diagnosis and a prognostic prediction of renal cell carcinoma. The method of the invention is useful in a primary screening of renal cell carcinoma because it allows testing of many people at once.

## Claims

1. A method for determining the presence of renal cell carcinoma in a patient comprising the following steps:
a) collecting all N-linked sugar chains from serum of the patient;
b) obtaining a quantitive profile of said N-linked sugar chains;
c) calculating an expression level of one sugar chain as detected, a ratio of expression level of one sugar chain to another sugar chain as detected or a summation of expression levels of sugar chains as detected; and
d) determining the presence of renal cell carcinoma utilizing said expression level, said ratio of expression level or said summation of expression levels as an index of the determination.

2. The method according to claim 1 wherein the sugar chain(s) in step (c) is selected from the group consisting of RC1, RC5, RC15, RC19, RC22, RC23, RC25, RC32, RC33, RC47 and a sugar chain having a tetra-antennary structure or a tri-antennary and bisecting structure.

3. The method according to claim 1 wherein an expression level of RC33 or RC19 is calculated in step (c).

4. The method according to claim 1 wherein a ratio of expression level of RC33 to RC15 (RC33/RC15), RC33 to RC1 (RC33/RC1), RC47 to RC15 (RC47/RC15), RC33 to RC22 (RC33/RC22), RC19 to RC15 (RC19/RC15), RC19 to RC32 (RC19/RC32), RC1 to RC19 (RC1/RC19), RC33 to RC5 (RC33/RC5), RC23 to RC5 (RC23/RC5) or RC33 to RC25 (RC33/RC25) is calculated in step (c).

5. The method according to claim 1 wherein a summation of expression levels of sugar chains having a tetra-antennary structure or a tri-antennary and bisecting structure is calculated in step (c).

6. A method for determining the degree of malignancy of renal cell carcinoma in a patient comprising the following steps:
a) collecting all N-linked sugar chains from serum of the patient;
b) obtaining a quantitive profile of said N-linked sugar chains;
c) calculating a ratio of expression level of one sugar chain to another sugar chain as detected; and
d) determining the degree of malignancy of renal cell carcinoma utilizing said ratio of expression level as an index of the determination.

7. The method according to claim 6 wherein the sugar chain(s) in step (c) is selected from the group consisting of RC2, RC3, RC4, RC5, RC6, RC7, RC8, RC9, RC12, RC13, RC17, RC18, RC19, RC29, RC31 and RC40.

8. The method according to claim 6 or 7 wherein the degree of malignancy of renal cell carcinoma is determined as to whether it is advanced renal cell carcinoma or not.

9. The method according to claim 8 wherein the ratio of expression level of RC31 to RC29 (RC31/RC29), RC9 to RC6 (RC9/RC6) or RC4 to RC6 (RC4/RC6) is calculated in step (c).

10. The method according to claim 6 or 7 wherein the degree of malignancy of renal cell carcinoma is determined as to the prognosis of said renal cell carcinoma.

11. The method according to claim 10 wherein the ratio of expression level of RC3 to RC6 (RC3/RC6), RC7 to RC8 (RC7/RC8), RC2 to RC8 (RC2/RC8), RC17 to RC18 (RC17/RC18), RC12 to RC13 (RC12/RC13), RC3 to RC8 (RC3/RC8), RC5 to RC29 (RC5/RC29), RC5 to RC40 (RC33/RC5) or RC19 to RC5 (RC19/RC5) is calculated in step (c).

12. Use of a sugar chain selected from the group consisting of RC1, RC5, RC15, RC19, RC22, RC23, RC25, RC32, RC33 and RC47 or a sugar chain having a tetra-antennary structure or a tri-antennary and bisecting structure as a diagnostic marker of renal cell carcinoma.

13. Use according to claim 12 wherein the selected sugar chain is RC33 or RC19.

14. Use according to claim 12 wherein the sugar chains are selected as the combination of RC33 and RC15, RC33 and RC1, RC47 and RC15, RC33 and RC22, RC19 and RC15, RC19 and RC32, RC1 and RC19, RC33 and RC5, RC23 and RC5 or RC33 and RC25.

15. Use according to claim 12 wherein the selected sugar chain is a sugar chain having a tetra-antennary structure or a tri-antennary and bisecting structure.

16. Use of a sugar chain selected from the group consisting of RC2, RC3, RC4, RC6, RC7, RC8, RC9, RC12, RC13, RC17, RC18, RC29 and RC31 as a diagnostic marker of advanced renal cell carcinoma.

17. Use according to claim 16 wherein the sugar chains are selected as the combination of RC31 and RC29, RC9 and RC6 or RC4 and RC6.

18. Use of a sugar chain selected from the group consisting of RC2, RC3, RC4, RC5, RC6, RC7, RC8, RC9, RC12, RC13, RC17, RC18, RC19, RC29, RC31 and RC40 as a predictive marker for prognosis of renal cell carcinoma.

19. Use according to claim 18 wherein the sugar chains are selected as the combination of RC3 and RC6, RC7 and RC8, RC2 and RC8, RC17 and RC18, RC12 and RC13, RC3 and RC8, RC5 and RC29, RC5 and RC40 or RC19 and RC5.
